# DEMANDE DE BREVET EUROPEEN

(11) **EP 1 698 337 A1**
(43) Date de publication de la demande: **06.09.2006**
(21) Numéro de dépôt: 06300161.4
(22) Date de dépôt: 24.02.2006
(51) Int. Cl.: A61K 31/445, A61K 8/49, A61Q 7/00, A61P 17/14

(54) **Utilisation capillaire de dérivés aminés cycliques**

(30) Priorité: 24.02.2005 FR 0550512
(71) Demandeur: L'ORÉAL, 75008 Paris (FR)
(72) Inventeur: Cavezza, Alexandre, 93320, Les Pavillons sous bois (FR)
(74) Mandataire: Le Coupanec, Pascale A.M.P.

(57) **Abrégé**

La présente invention concerne l'utilisation cosmétique d'au moins un dérivé de pipéridine de formule (I) ou de l'un de ses sels ou isomères, comme agent pour induire et/ou stimuler la croissance des fibres kératiniques des êtres humains et/ou freiner leur chute et/ou augmenter leur densité, 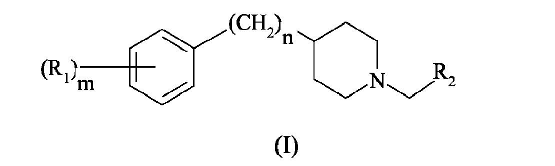 dans laquelle :
- R₁ désigne un halogène ou un radical choisi parmi un radical alkyle linéaire ou ramifié en C₁-C₆, un groupe OR, un groupe NRR', un groupe CF₃, un groupe NHCOR ou un groupe CONRR' ;
- R₂ désigne un radical alkyle ou alkényle linéaire ou ramifié, en C₁-C₂₀, dont la chaîne hydrocarbonée peut être éventuellement interrompue par une fonction - CO- éventuellement substitué par au moins un groupe OR, COOR, NRR', NHCOR ou CONRR' et/ou par un groupe phényle éventuellement substitué par un ou plusieurs radicaux R₁ ;
avec R et R' désignant, indépendamment l'un de l'autre, un atome d'hydrogène ou un radical alkyle en C₁-C₆, linéaire ou ramifié,
- m représente un entier variant de 0 à 5 ; et
- n représente un entier variant de 0 à 5.

## Description

La présente invention concerne plus particulièrement l'utilisation d'une famille de dérivés aminés cycliques notamment comme actif anti-chute de cheveux et/ou pour traiter l'alopécie.

La croissance des cheveux et leur renouvellement sont principalement déterminés par l'activité des follicules pileux et de leur environnement matriciel. Cette activité est cyclique et comporte essentiellement trois phases à savoir la phase anagène, la phase catagène et la phase télogène.

La phase anagène (phase active ou de croissance), qui dure plusieurs années correspond à la phase active ou de croissance des cheveux.

La phase catagène, qui succède à la phase anagène, est en revanche très courte, transitoire et dure quelques semaines. Au cours de cette phase, le cheveu subit une évolution, le follicule s'atrophie et son implantation dermique apparaît de plus en plus haute.

La phase terminale ou phase télogène, qui dure quelques mois, correspond à une phase de repos du follicule et le cheveu finit par tomber. A la fin de cette période de repos, un nouveau follicule est régénéré, sur place, et un autre cycle recommence.

La chevelure se renouvelle donc en permanence et sur les 150 000 cheveux environ que comporte une chevelure, 10 % environ sont au repos et seront remplacés en quelques mois. La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins et leurs cycles plus courts.

A l'âge adulte, le système vasculaire de la peau est parachevé et ne se modifie plus sauf au niveau des follicules pileux où il subit d'importantes variations à chaque cycle pilaire. Les follicules pileux sont en effet une structure cutanée richement innervée et très vascularisée. Le phénomène de développement de la microcirculation des follicules pileux est appelé angiogénèse. Au début de chaque phase anagène, il est nécessaire de développer une forte activation de l'angiogénèse afin de re-développer le microréseau vasculaire périfolliculaire. L'involution de ce microréseau et la disparition des capillaires sanguins de la papille dermique vont de pair avec le changement de phase et le passage en phase catagène. A ce stade, les microvaisseaux sanguins collapsent et disparaissent.

Parallèlement, dans les zones alopéciques, une fibrose périfolliculaire s'installe, les follicules se miniaturisent cycle après cycle et progressivement, la vascularisation spécifique des bulbes s'amoindrie.

Le phénomène d'angiogénèse observé au cours de la phase anagène dépend de nombreux facteurs trophiques, de cytokines ou d'autres molécules biologiquement actives apportées par la circulation sanguine ou localement produites en particulier par les fibroblastes de la papille dermique ou les kératinocytes du bulbe capillaire. Parmi ces facteurs trophiques on peut citer le facteur de croissance des cellules endothéliales (appelé également vascular endothelial growth factor (VEGF) en terminologie anglo-saxonne). Ce facteur est essentiel pour l'angiogénèse et augmente la perméabilité vasculaire. Des études ont montré que l'expression de ce facteur était augmentée au cours de la phase anagène du cycle pilaire. Ainsi, ce facteur contribue au maintien d'une microvascularisation fonctionnelle autour du follicule pileux, et notamment de la base du bulbe et de la papille dermique, ainsi qu'à l'apport de nutriments nécessaires à la bonne croissance du cheveu.

La microcirculation périfolliculaire joue donc un rôle primordial dans le processus de croissance pilaire en apportant les facteurs et les nutriments nécessaires à la croissance de ce follicule.

En conséquence, toute altération de la microcirculation périfolliculaire entraînera une diminution de l'apport des éléments nutritifs et gazeux (oxygène notamment) nécessaires à la croissance pilaire conduisant à des troubles de la croissance du cheveu et la mise en place progressive d'une alopécie.

Ainsi, la chute des cheveux peut être fortement accentuée et les cycles de renouvellement des follicules peuvent être fortement perturbés dans certaines dermatoses du cuir chevelu à caractéristique inflammatoire, telles que par exemple le psoriasis ou les dermatites séborrhéiques.

Par ailleurs, une grossesse (post partum), des états de dénutrition ou malnutrition, de stress physiologique, de déséquilibres alimentaires ou encore des états d'asthénie ou de dysfonctionnement hormonal comme cela peut être le cas au cours ou au décours de la ménopause peuvent également entraîner une perte et/ou une altération importante, temporaire ou définitive, des cheveux. Il peut également s'agir de chute ou d'altérations des cheveux en relation avec des phénomènes saisonniers.

Il peut s'agir également d'une alopécie, qui est essentiellement due à une perturbation du renouvellement capillaire entraînant dans un premier temps l'accélération de la fréquence des cycles au détriment de la qualité des cheveux, puis de leur quantité. Il se produit alors un appauvrissement progressif de la chevelure et une miniaturisation progressive des cheveux, conjointement à un isolement des bulbes par une progression de l'épaisseur de la matrice collagénique périfolliculaire et de la gaine conjonctive externe. La revascularisation est donc rendue plus difficile cycle après cycle. Les cycles de croissance successifs aboutissent à des cheveux de plus en plus fins et de plus en plus courts, se transformant peu à peu en un duvet non pigmenté. Des zones sont touchées préférentiellement, notamment les golfes temporaux ou frontaux chez l'homme et, chez la femme, on constate une alopécie diffuse du vertex.

Le terme alopécie recouvre aussi toute une famille d'atteintes du follicule pileux ayant pour conséquence finale la perte définitive, partielle ou générale des cheveux. Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.

On recherche depuis de nombreuses années, dans l'industrie cosmétique ou pharmaceutique, des compositions permettant de supprimer ou de réduire l'alopécie, et notamment d'induire ou de stimuler la croissance des cheveux ou de diminuer leur chute. L'une des voies explorées est plus particulièrement celle de la vascularisation autour du follicule pileux.

De façon générale, tout facteur entraînant une augmentation de l'apport sanguin au niveau du follicule pileux soit en activant l'angiogénèse, soit en s'opposant à sa régression, soit encore en agissant sur les microvaisseaux pour limiter leur constriction, aura un effet bénéfique sur l'apport énergétique nécessaire à la bonne croissance de ce même follicule.

Un mode de contrôle d'une activité antichute/repousse des fibres kératiniques chez un composé vise précisément à tester l'efficacité de celui-ci pour la décontraction des muscles. L'homme de l'art sait que les effets consécutifs à cette décontraction, à savoir une réduction de l'épaisseur de la matrice collagénique autour des bulbes capillaires et/ou une augmentation de la vascularisation autour des bulbes capillaires sont prédictifs d'un effet bénéfique du composé testé en termes d'action antichute et/ou repousse des fibres kératiniques.

Ainsi, un des composés connu pour maintenir la vascularisation périfolliculaire est le vérapamil, qui est un antagoniste puissant des canaux calciques de type L. Le vérapamil et d'autres antagonistes de canaux calciques comme le diltiazem et la nifédipine sont décrits comme étant actifs dans le traitement de la chute des cheveux, en particulier par leurs effets sur la microcirculation (confère les documents de Shiseido JP88/062680 et de Coppe J. BE/89/000305).

En outre, il existe des documents décrivant l'utilisation de donneurs de NO (monoxyde d'azote) pour application sur le cuir chevelu, pour stimuler la croissance de cheveux en agissant sur la microcirculation du cuir chevelu. Ainsi, le document brevet de Proctor (EP 0 327 263) décrit l'utilisation de composés producteurs du radical NO, en association avec des agents réducteurs, des anti-oxydants et avec des piégeurs de radical hydroxyle. Un autre document brevet de E. Fossel (WO 99/13717) décrit l'utilisation de l'arginine et de ses dérivés, comme substrat de NO-synthase, pour la formation in vivo de NO et leur utilisation (entre autre) dans le traitement de l'alopécie. Un autre document brevet de Shiseido (JP-A-07316023) décrit également l'utilisation de l'arginine et de ses dérivés dans le traitement de l'alopécie.

Ces substances connues présentent toutefois des effets indésirables. En particulier, elles présentent des activités multiples, pouvant perturber l'équilibre ionique et physiologique des cellules cutanées. Autrement dit, leur activité multiple rend difficile leur contrôle d'action sur les cellules.

Plus récemment, la demande EP 1 506 767 a décrit une famille particulière d'amines carbonylés présentant un effet bénéfique en terme d'amélioration de la vascularisation du follicule pileux, privilégiant ainsi la pousse des cheveux.

La présente invention résulte plus particulièrement de l'observation par les inventeurs qu'une famille particulière de dérivés pipéridine possédait également une activité bénéfique à l'égard des follicules pileux, de leur région périphérique et/ou des cheveux.

Parmi les composés utilisés selon l'invention, certains ont déjà été décrits comme inhibiteurs de canaux calciques dans les demandes EP-0 542 846 et JP-61 027 963 et dans le brevet US-4,952,560.

D'autres dérivés de pipéridine ont été décrits comme agents anti-bactériens (EP-0 308 328).

La demande JP 62 270 514 a proposé pour sa part d'utiliser un dérivé pipéridinique spécifique, l'Ifenprodil, dans des compositions capillaires pour améliorer la circulation sanguine du cuir chevelu.

En conséquence, selon un premier aspect, la présente invention concerne l'utilisation notamment cosmétique, comme agent pour induire et/ou stimuler la croissance des fibres kératiniques des êtres humains notamment les cils et les cheveux et/ou freiner leur chute et/ou augmenter leur densité, d'au moins un dérivé de pipéridine de formule (I) : 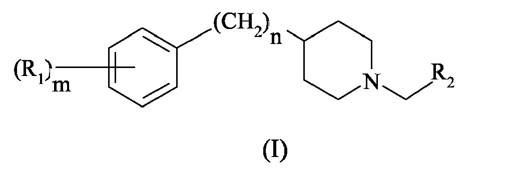 dans laquelle :
- R₁ désigne un halogène ou un radical choisi parmi un radical alkyle linéaire ou ramifié en C₁-C₆, un groupe OR, un groupe NRR', un groupe CF₃, un groupe NHCOR ou un groupe CONRR' ;
- R₂ désigne un radical alkyle ou alkényle linéaire ou ramifié, en C₁-C₁₉, dont la chaîne hydrocarbonée peut être éventuellement interrompue par une fonction -CO-et éventuellement substituée par au moins un groupe OR, COOR, NRR', NHCOR ou CONRR' et/ou par un groupe phényle éventuellement substitué par un ou plusieurs radicaux R₁,
   avec R et R' désignant, indépendamment l'un de l'autre, un atome d'hydrogène ou un radical alkyle en C₁-C₆, linéaire ou ramifié,
- m représente un entier variant de 0 à 5 ;
- n représente un entier variant de 0 à 5 ; et
- leurs sels et isomères.

Selon un autre de ses aspects, l'invention a encore pour objet l'utilisation d'au moins une dérivé pipéridine de formule générale (I) ou de l'un de ses sels ou isomères, pour la préparation d'une composition cosmétique de soin et/ou de traitement des fibres kératiniques d'êtres humains, destinée à réduire la chute des fibres kératiniques et/ou augmenter leur densité.

Selon un autre de ses aspects, l'invention a encore pour objet l'utilisation d'au moins une dérivé pipéridine de formule générale (I) ou de l'un de ses sels ou isomères, pour la préparation d'une composition cosmétique de soin et/ou de traitement des fibres kératiniques d'êtres humains, destinée à induire et/ou stimuler la croissance des fibres kératiniques.

Les fibres kératiniques humaines auxquelles s'applique l'invention sont notamment les cheveux, les ongles, les sourcils, les cils, les poils de barbe, de moustache et les poils pubiens. Plus spécialement, l'invention s'applique aux cheveux et/ou aux cils humains.

En particulier, l'invention se rapporte encore à l'utilisation cosmétique d'au moins un dérivé de pipéridine de formule générale (I) ou de l'un de ses sels ou isomères, pour la préparation d'une composition cosmétique de soin capillaire d'êtres humains déstinée à traiter l'alopécie andro-chrono-génétique.

Elle a encore pour objet l'utilisation d'au moins un dérivé de pipéridine de formule générale (I) ou d'un de ses sels ou isomères pour la préparation d'une composition capillaire pour êtres humains, destinée à induire et/ou stimuler la croissance des cheveux. En particulier, cette composition permet de maintenir en bon état la chevelure et/ou de lutter contre la chute naturelle des cheveux plus spécialement des hommes.

L'invention a encore pour objet l'utilisation cosmétique d'au moins un dérivé de pipéridine de formule générale (I) ou d'un de ses sels ou isomères, pour la préparation d'une composition de soin et/ou de maquillage des cils d'êtres humains, destinée à induire et/ou stimuler la croissance des cils et/ou augmenter leur densité. Cette composition permet ainsi de maintenir en bon état les cils et/ou améliorer leur état et/ou leur aspect.

L'invention a encore pour objet l'utilisation d'au moins un dérivé de pipéridine de formule générale (I) ou d'un de ses sels ou isomères, pour la fabrication d'une composition destinée à traiter les désordres liés à une réduction de la microcirculation ou vascularisation cutanée et notamment du follicule pileux chez l'être humain.

Dans la formule (I), les groupes alkyle peuvent notamment être choisis, selon le cas, parmi les groupes : méthyle, éthyle, n-propyle, isopropyle, n-butyle, isobutyle, tert-butyle, pentyle, hexyle, heptyle, octyle, nonyle, décyle, undécyle, dodécyle, myristyle, palmityle, stéaryle et arachidyle.

En outre, dans le contexte de cette demande, on entend par "alkènyle" des radicaux pouvant comprendre une ou plusieurs doubles liaisons, conjuguées ou non. Ils peuvent notamment être choisis, selon le cas, parmi les groupes : vinyle, allyle, butényle ou pentényle.

Certains composés de formule générale (I) peuvent exister sous différentes formes isomériques telles que éniantomères, diastéréoisomères, isomères géométriques ou tautomères. La présente invention s'étend à l'ensemble de ces isomères et à leur mélange en toutes proportions.

Comme sels du composé de formule (I), on peut citer les sels obtenus par addition du composé de formule (I) avec un acide inorganique, choisi notamment parmi les acides chlorhydrique, sulfurique et phosphorique, ou avec un acide organique, choisi en particulier parmi les acides acétique, propionique, succinique, fumarique, maléique, lactique, glycolique, citrique et tartrique. Les sels incluent les sels métalliques tels que les sels alcalins ou alcalinoterreux.

Les composés selon l'invention peuvent également exister sous la forme de solvates et notamment hydrates qui font également partie du domaine de l'invention.

De préférence, le dérivé de pipéridine selon l'invention est tel que l'une au moins, et de préférence toutes, les conditions suivantes sont satisfaites :
- m varie de 0 à 3 et est de préférence égal à 0
- n est égal 0, 1, 2 ou 3, et de préférence à 0 ou 3,
- R₂ désigne un radical alkyle ou alkényle linéaire ou ramifié, en C₁-C₁₄, dont la chaîne hydrocarbonée peut être interrompue par un motif carbonyle et/ou être substituée par un groupe phényle et/ou une fonction hydroxyle.

S'avèrent tout particulièrement avantageux dans le cadre de la présente invention, les composés suivants : 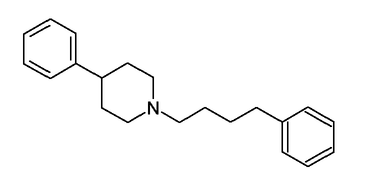 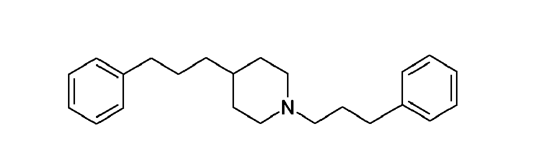 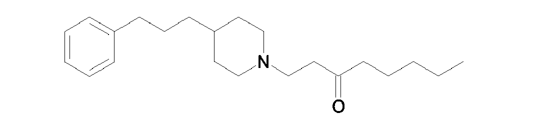 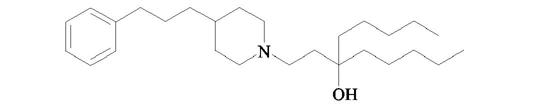 leurs sels, isomères et leurs mélanges.

Les composés de formules (I) peuvent notamment être préparés selon le schéma réactionnel suivant : 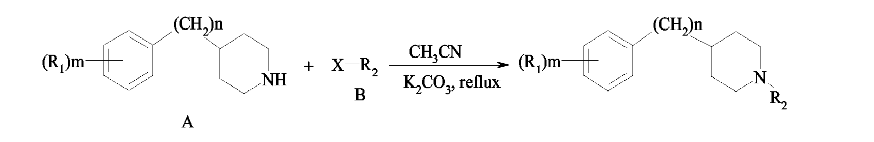 par réaction d'un équivalent de pipéridine substituée A avec un équivalent de B, où X désigne un groupe partant de type halogène ou sulfonate, en présence de K₂CO₃ dans l'acétonitrile à reflux pendant une nuit. Le produit obtenu peut être traité et purifié sur colonne de silice.

La quantité de dérivé de pipéridine de formule générale (I) ou (II) utilisable selon l'invention est bien entendu fonction de l'effet recherché et peut donc varier dans une large mesure.

La quantité efficace correspond à la quantité nécessaire pour obtenir le résultat désiré (en particulier à savoir augmenter la densité des fibres kératiniques, inhiber la chute et/ou favoriser leur pousse). L'homme du métier est donc en mesure d'évaluer cette quantité efficace qui dépend de la nature de l'amine utilisée, de la personne à laquelle on l'applique, et du temps de cette application.

Pour donner un ordre de grandeur, on peut utiliser ces dérivés en une quantité représentant de 0,001 % à 10 % du poids total de la composition, préférentiellement en une quantité représentant de 0,01 % à 5 % du poids total de la composition, plus préférentiellement en une quantité représentant de 0,1 % à 2 % du poids total de la composition.

Dans la suite du texte, et sauf indication contraire, les quantités des différents ingrédients de la composition sont données en pourcentage en poids par rapport au poids total de la composition.

Les compositions de l'invention peuvent être à usage cosmétique ou pharmaceutique (notamment dermopharmaceutique). Préférentiellement, les compositions de l'invention sont à usage cosmétique. Les compositions doivent contenir un milieu physiologiquement acceptable non toxique et susceptible d'être appliqué sur la peau, y compris le cuir chevelu et les paupières, et sur les fibres kératiniques comme les cheveux et les cils.

Les dérivés de formule (I), salifiée ou non, peuvent être utilisés dans une composition à ingérer, injecter ou appliquer sur la peau ou les fibres kératiniques (sur toute zone cutanée ou fibres à traiter).

Ces composés peuvent également être administrés par la voie orale en une quantité de 0,1 à 300 mg, et en particulier de 5 à 10 mg par jour.

Une composition préférée de l'invention est une composition à usage cosmétique et en particulier d'application topique sur la peau et les fibres kératiniques, et plus spécialement sur le cuir chevelu, les cheveux et les cils.

Cette composition peut se présenter sous toutes formes galéniques connues adaptées au mode d'utilisation.

Pour une application topique sur la peau et les fibres kératiniques, y compris le cuir chevelu, la composition peut avoir la forme d'une solution ou suspension aqueuse, alcoolique, hydro-alcoolique ou huileuse, d'une émulsion ou dispersion de consistance plus ou moins fluide et notamment liquide ou semi-liquide, obtenue par dispersion d'une phase grasse dans une phase aqueuse (H/E) ou inversement (E/H), d'une dispersion ou émulsion solide (H/E) ou (E/H), d'un gel aqueux, hydro-alcoolique ou huileux plus ou moins fluide ou solide, d'une poudre libre ou compactée à utiliser telle quelle ou à incorporer dans un milieu physiologiquement acceptable, ou encore de microcapsules ou microparticules, de dispersions vésiculaires de type ionique et/ou non ionique.

On peut également envisager une composition sous forme de mousse ou encore sous forme de spray ou d'aérosol comprenant alors un agent propulseur sous pression.

La composition peut ainsi se présenter sous forme d'une lotion, sérum, lait, crème H/E ou E/H, gel, onguent, pommade, poudre, baume, patch, tampon imbibé, savon, pain, mousse.

En particulier la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application sur les cils ou les poils, la composition à laquelle s'applique l'invention peut se présenter sous forme d'un mascara, pigmenté ou non, à appliquer à la brosse sur les cils ou encore sur les poils de barbe ou de moustache.

Pour une application sur les ongles, les composés conformes à l'invention, peuvent être formulés sous l'aspect d'une composition de type vernis.

Pour une composition à usage par injection, la composition peut se présenter sous forme de lotion aqueuse ou de suspension huileuse par exemple sous forme de sérum. Pour un usage par voie orale, la composition peut se présenter sous forme de capsules, de granulés, de sirops buvables ou de comprimés.

Selon un mode de réalisation particulier, la composition selon l'invention se présente sous forme de crème ou lotion capillaire, de shampooing ou d'après-shampooing capillaire, de mascara capillaire ou pour cils.

Les quantités des différents constituants du milieu physiologique de la composition selon l'invention sont celles généralement utilisées dans les domaines considérés. En outre, ces compositions sont préparées selon les méthodes usuelles.

Lorsque la composition est une émulsion, la proportion de la phase grasse peut aller de 2 % à 80 % en poids, et de préférence de 5 % à 50 % en poids par rapport au poids total de la composition. La phase aqueuse est ajustée en fonction de la teneur en phase grasse et en composé(s) (I) ainsi que de celle des éventuels ingrédients additionnels, pour obtenir 100 % en poids. En pratique la phase aqueuse représente de 5 % à 99,9 % en poids.

La phase grasse peut contenir des composés gras ou huileux, liquides à température ambiante (25 °C) et pression atmosphérique (760 mm de Hg), généralement appelés huiles. Ces huiles peuvent être compatibles ou non entre elles et former une phase grasse liquide macroscopiquement homogène ou un système bi- ou tri-phasique. En plus des ces huiles, la phase grasse peut, contenir des cires, des gommes, des polymères lipophiles, des produits "pâteux" ou visqueux contenant des parties solides et des parties liquides.

La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.

Les émulsionnants et coémulsionnants utilisés pour l'obtention d'une composition sous forme d'émulsion sont ceux généralement utilisés dans les domaines cosmétique et pharmaceutique. Leur nature est, en outre, fonction du sens de l'émulsion. En pratique, l'émulsionnant et éventuellement le coémulsionnant sont présents, dans la composition, en une proportion allant de 0,1 % à 30 % en poids, de préférence de 0,5 à 20 % en poids et mieux de 1 à 8 %. L'émulsion peut, en outre, contenir des vésicules lipidiques et notamment des liposomes.

Lorsque la composition est sous forme d'une solution ou d'un gel huileux, la phase grasse peut représenter plus de 90 % du poids total de la composition.

Avantageusement, pour une application capillaire, la composition est une solution ou suspension aqueuse, alcoolique ou hydro-alcoolique et mieux une solution ou suspension eau/éthanol. La fraction alcoolique peut représenter de 5 % à 99,9 % et mieux de 8 % à 80 %.

Pour une application mascara, la composition est en particulier une dispersion de cire-dans-eau ou de cire-dans-huile, une huile gélifiée, un gel aqueux. Elle peut être pigmentée ou non.

La composition de l'invention peut comprendre, en outre, d'autres ingrédients usuellement utilisés dans les domaines concernés, choisis parmi les solvants, les épaississants ou gélifiants de phase aqueuse ou de phase huileuse, les matières colorantes solubles dans le milieu de la composition, les particules solides du type charges ou pigments, les antioxydants, les conservateurs, les parfums, les électrolytes, les neutralisants, les agents bloqueurs d'U.V. comme les filtres solaires, les polymères filmogènes, les actifs cosmétiques et pharmaceutiques à action bénéfique pour la peau ou les fibres kératiniques, autres que les composés de formule (I) ou (II) (comme les vitamines), leurs mélanges. Ces additifs peuvent être présents dans la composition selon les quantités généralement utilisées dans le domaine cosmétique et dermatologique et notamment à raison de 0,01 à 50 % du poids total de la composition et mieux de 0,1 à 20 % et par exemple de 0,1 à 10 %. Ces adjuvants, selon leur nature, peuvent être introduits dans la phase grasse, dans la phase aqueuse et/ou dans les vésicules lipidiques et notamment des liposomes.

Bien entendu l'homme du métier veillera à choisir les éventuels additifs complémentaires et/ou leur quantité de telle manière que les propriétés avantageuses de la composition selon l'invention, notamment l'augmentation de la densité des fibres kératiniques, ne soient pas ou substantiellement pas, altérées par l'adjonction envisagée.

Comme solvants utilisables dans l'invention, on peut citer les alcools inférieurs en C₂ à C₈ comme l'éthanol, l'isopropanol, le propylène glycol et certaines huiles cosmétiques légères comme les alcanes en C₆ à C₁₆.

Comme huiles utilisables dans l'invention, on peut citer les huiles d'origine minérale (huile de vaseline, isoparaffine hydrogénée), les huiles d'origine végétale (fraction liquide du beurre de karité, huile de tournesol, d'abricot, de soja, alcool ou acide gras), les huiles d'origine animale (perhydrosqualène), les huiles de synthèse (esters d'acide gras, huile de Purcellin), les huiles siliconées (polydiméthylsiloxanes linéaires ou cycliques, phényltriméthicones) et les huiles fluorées (perfluoropolyéthers). Comme cires, on peut citer les cires siliconées, les cires d'abeille, de candellila, de riz, de carnauba, de paraffine ou de polyéthylène.

Comme émulsionnants utilisables dans l'invention, on peut citer par exemple le stéarate ou laurate de glycérol, les stéarates ou oléates de sorbitol, les alkyl diméthiconecopolyol (avec alkyle ≥ 8) et leurs mélanges pour une émulsion E/H. On peut aussi utiliser le monostéarate ou monolaurate de polyéthylène glycol, le stéarate ou oléate de sorbitol polyoxyéthyléné, les diméthiconecopolyols et leurs mélanges pour une émulsion H/E. L'émulsionnant et le coémulsionnant sont présents, dans la composition, en une proportion allant de 0,3 à 30 % en poids, et de préférence de 0,5 à 20 % en poids par rapport au poids total de la composition.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et comme gélifiants lipophiles, on peut citer les argiles modifiées comme les Bentones, les sels métalliques d'acides gras comme les stéarates d'aluminium, la silice traitée hydrophobe, l'éthylcellulose, leurs mélanges.

Comme actif cosmétique ou pharmaceutique autre que les composés de formule (I) ou (II), la composition peut contenir un actif additionnel hydrophile choisi parmi les protéines ou les hydrolysats de protéine, les acides aminés, les polyols, l'urée, l'allantoïne, les sucres et les dérivés de sucre, les vitamines hydrosolubles, les extraits végétaux (ceux d'Iridacées ou de soja) et les hydroxy-acides (acide de fruit, acide salicylique) ; et/ou un actif additionnel lipophile choisi parmi le rétinol (vitamine A) et ses dérivés notamment ester (palmitate de rétinol), le tocophérol (vitamine E) et ses dérivés notamment ester (acétate ou palmitate de tocophérol), les acides gras essentiels, les céramides, les huiles essentielles, les dérivés de l'acide salicylique comme le n-octanoyl-5 salicylique, les esters des hydroxy acides, les phospholipides comme la lécithine, leurs mélanges.

Selon un mode particulier de réalisation de l'invention, on peut associer au composé de formule générale (I) ou à l'un de ses sels ou isomères, au moins un composé additionnel favorisant la repousse et/ou limitant la chute des fibres kératiniques (cheveux ou cils). Ces composés additionnels sont notamment choisis parmi les inhibiteurs de lipoxygénase tels que décrits dans EP 0 648 488, les inhibiteurs de bradykinine décrits notamment dans EP 0 845 700, les prostaglandines et leurs dérivés notamment ceux décrits dans WO 98/33497, WO 95/11003, JP 97-100091, JP 96-134242, les agonistes ou antagonistes des récepteurs des prostaglandines, les analogues non prostanoïques de prostaglandines tels que décrits dans EP 1 175 891 et EP 1 175 890, WO 01/74307, WO 01/74313, WO 01/74314, WO 01/74315 ou WO 01/72268, leurs mélanges.

Comme autres composés additionnels favorisant la pousse du cheveu pouvant être présents dans la composition selon l'invention on peut citer les vasodilatateurs, les antiandrogènes, les cyclosporines et leurs analogues, les antimicrobiens et antifongiques, les anti-inflammatoires, les rétinoïdes, seuls ou en mélange.

Les vasodilatateurs utilisables sont notamment les agonistes des canaux potassium incluant le minoxidil ainsi que les composés décrits dans les brevets US 3 382 247, 5 756 092, 5 772 990, 5 760 043, 5 466 694, 5 438 058, 4 973 474, la cromakalim, le nicorandil et le diaxozide, seuls ou en association.

Les antiandrogènes utilisables incluent notamment les inhibiteurs stéroïdiens ou non stéroïdiens de 5α-réductase, comme le finastéride et les composés décrits dans le document US 5 516 779, l'acétate de cyprostérone, l'acide azélaïque, ses sels et ses dérivés et les composés décrits dans US 5 480 913, le flutamide, l'oxendolone, la spironolactone, le diéthylstilbestrol et les composés décrits dans les brevets US 5 411 981, 5 565 467 et 4 910 226.

Les composés antimicrobiens ou antifongiques peuvent être choisis parmi les dérivés du sélénium, l'octopirox, le triclocarban, le triclosan, le pyrithione zinc, l'itraconazole, l'acide asiatique, l'hinokitiol, la mipirocine, les tétracyclines, notamment l'érythromycine et les composés décrits dans EP 0 680 745, le chlorhydrate de clinycine, le peroxyde de benzoyle ou de benzyle, la minocycline et les composés appartenant à la classe des imidazoles tels que l'éconazole, le kétoconazole ou le miconazole ou leurs sels, les esters d'acide nicotinique, dont notamment le nicotinate de tocophérol, le nicotinate de benzyle et les nicotinates d'alkyles en C₁-C₆ comme les nicotinates de méthyle ou d'hexyle.

Les anti-inflammatoires peuvent être choisis parmi les anti-inflammatoires stéroïdiens comme les glucocorticoïdes, les corticostéroïdes (par exemple : l'hydrocortisone) et les anti-inflammatoires non stéroïdiens comme l'acide glycyrrhétinique et l'α-bisabolol, la benzydamine, l'acide salicylique et les composés décrits dans EP 0 770 399, WO 94/06434 et FR 2 268 523.

Les rétinoïdes peuvent être choisis parmi l'isotrétinoïne, l'acitrétine et le tazarotène.

Comme autres composés additionnels actifs pour favoriser la pousse et/ou limiter la chute des cheveux utilisables en association avec le composé de formule (I), salifié ou non, on peut citer l'aminexil, le 6-0-[(9Z,12Z)-octadéca-9,12-diènoyl]hexapyranose, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les benzophénones et l'hydantoïne, l'acide rétinoïque ; les vitamines comme la vitamine D, les analogues de la vitamine B12 et le panthoténol ; les triterpènes comme l'acide ursolique et les composés décrits dans US 5 529 769, US 5 468 888, US 5 631 282 ; les agents antiprurigineux comme la thénaldine, la triméprazine ou la cyproheptadine ; les antiparasitaires, en particulier le métronidazole, le crotamiton ou les pyréthrinoïdes ; les agents antagonistes de calcium, comme la cinnarizine, le diltiazem, la nimodipine, vérapamil, l'alvérine et la nifédipine ; les hormones telles que l'estriol ou ses analogues, la thyroxine et ses sels, la progestérone ; les antagonistes du récepteur FP (récepteur aux prostaglandines du type F) tels que le latanoprost, le bimatoprost, le travoprost, l'unoprostone ; les inhibiteurs de 15-hydroxyprostaglandine désydrogénase ; leurs mélanges.

On peut également envisager que la composition comprenant au moins un composé de formule générale (I), salifié ou non, soit sous forme liposomée, telle que notamment décrite dans le document WO 94/22468. Ainsi, le composé encapsulé dans les liposomes peut être délivré sélectivement au niveau du follicule pileux.

La composition à laquelle s'applique l'invention peut être appliquée sur les zones alopéciques du cuir chevelu et des cheveux d'un individu, et éventuellement laissée en contact plusieurs heures et éventuellement rincée.

On peut, par exemple, appliquer la composition contenant une quantité efficace d'un composé de formule générale (I), salifiée ou non, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des fibres kératiniques humaines et/ou de la peau d'où émergent ces fibres dont le cuir chevelu et les paupières, destiné à stimuler la croissance des fibres kératiniques humaines telles que les cheveux et les cils d'être humain et/ou freiner leur chute,
caractérisé par le fait qu'il comprend au moins l'application sur les fibres kératiniques humaines et/ou la peau d'où émergent les fibres, d'une composition cosmétique comprenant une quantité efficace d'au moins un composé de formule (I) ou d'un de ses sels ou isomères, à laisser celle-ci en contact avec les fibres kératiniques et/ou la peau, et éventuellement le rinçage des fibres kératiniques et/ou la peau.

Ce procédé de traitement présente les caractéristiques d'un procédé cosmétique dans la mesure où il permet d'améliorer l'esthétique des fibres kératiniques et en particulier des cheveux et des cils en leur donnant une plus grande vigueur et un aspect amélioré. En outre, il peut être utilisé quotidiennement pendant plusieurs mois, sans prescription médicale.

Plus spécialement, la présente invention a pour objet un procédé de soin cosmétique des cheveux et/ou du cuir chevelu humains, en vue d'améliorer leur état et/ou leur aspect, caractérisé en ce qu'il consiste à appliquer sur les cheveux et/ou le cuir chevelu, une composition cosmétique comprenant au moins un composé de formule générale (I) ou l'un de ses sels ou isomères, à laisser celle-ci au contact des cheveux et/ou du cuir chevelu, et éventuellement à rincer les cheveux et/ou le cuir chevelu.

L'invention a encore pour objet un procédé de soin cosmétique et/ou de maquillage ces cils humains, notamment en vue d'améliorer leur état et/ou leur aspect, caractérisé en ce qu'il comprend au moins l'application sur les cils et/ou les paupières d'une composition de mascara comprenant au moins un composé de formule générale (I) ou l'un de ses sels ou isomères. Cette composition de mascara peut être appliquée seule ou en sous-couche d'un mascara pigmenté classique et être éliminée comme un mascara pigmenté classique.

Avantageusement, dans le procédé selon l'invention, on applique sur les zones à traiter du cuir chevelu entre 5 et 500 µl d'une solution ou composition telle que définie précédemment, comprenant de 0,001 % à 5 % en poids de composé de formule générale (I).

D'autres caractéristiques et avantages de l'invention ressortiront mieux des exemples qui suivent, donnés à titre illustratif et non limitatif. Dans ce qui suit, les proportions sont données en pourcentage massique, sauf indication contraire.

### Exemple 1

Les composés présentés dans le tableau I ont été préparés selon les protocoles discutés ci-après.

| | |
|---|---|
| 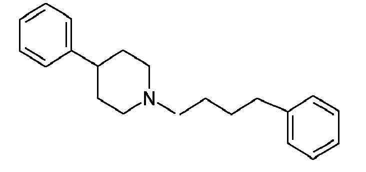 | Composé 1 |
| 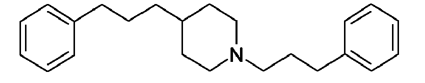 | Composé 2 |
| 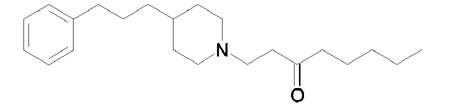 | Composé 3 |
| 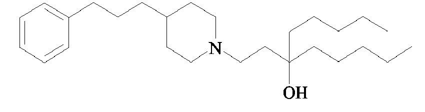 | Composé 4 |

### 1) Synthèse des composés 1 et 2

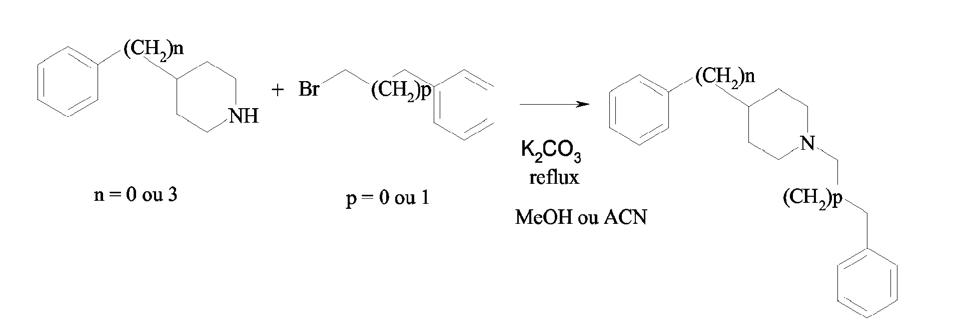

La pipérazine correspondante est mise en présence du bromo alkyle correspondant (1eq) en présence de K₂CO₃ dans le méthanol ou l'acétonitrile à reflux pendant une nuit. A l'issue de la réaction, le produit attendu est récupéré et purifié sur colonne de silice. Sa structure chimique est vérifiée par spectre RMN ¹H 500 MHz.

### 2) Synthèse du composé 3 et du composé 4

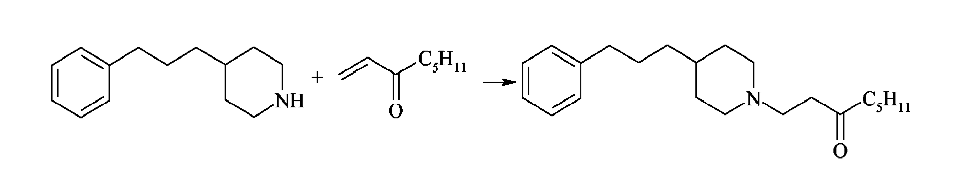

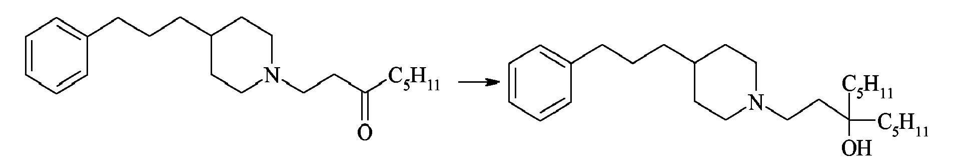

La 1-(3-phénylpropyl)-pipéridine (1eq) et la 1-octène-3-one (1eq) sont mises en présence dans l'éthanol à température ambiante. Le produit attendu (composé 3) est récupéré et purifié sur colonne de silice. Sa structure chimique est confirmée par analyses RMN et SM.

La cétone intermédiaire (1eq) correspondant au composé 3 est mise à réagir avec du bromopentyl magnésien (1eq) dans du THF anhydre sous azote, à la température ambiante. Le composé 4 ainsi obtenu est récupéré et purifié sur colonne de silice. Sa structure chimique est vérifiée par analyses RMN et SM.

### Exemple 2

Les composés 1 et 2 ont été testés à une concentration donnée, sur un modèle de coculture nerfs/muscles, qui permet de recréer un arc moteur en innervant des cellules musculaires striées humaines avec des explants de moelle épinière et de ganglions rachidiens d'embryons de rat.

Ce test est prédictif d'un effet inhibiteur des contractions des fibres musculaires.

Le protocole adopté est comme suit :

Les myoblastes humains (primoculture de cellules musculaires humaines, M1b) ont été ensemencés en plaques 24 puits. Le milieu de culture est un mélange de 2/3 MEM (Invitrogen 21090-022) 1/3 M199 (Invitrogen 31153-026) 5 % SVF. A confluence, les explants de moelle épinière d'embryons de rat de 13 jours sont déposés sur la primoculture. Les premières contractions des fibres musculaires sont observées après une semaine de coculture. Après 3 semaines, ces fibres musculaires sont striées et possèdent des jonctions neuromusculaires différenciées matures.

Les produits sont testés en triplicata (3 fibres analysées) à 2 concentrations. Pour chaque puits de culture, une fibre musculaire ayant des contractions régulières (> 60 contractions par minute) est sélectionnée et le nombre de contractions compté pendant 30 secondes. Le produit a testé est alors rajouté dans le milieu de culture sous microscope sans déplacer la platine. Après 60 secondes d'incubation, le nombre de contractions est à nouveau compté pendant 30 secondes. L'ensemble de l'expérimentation est enregistré à l'aide d'une caméra numérique. Les vidéos sont sauvegardées sur support informatique.

Le composé 1 induit un effet dose réponse avec une inhibition de 50 % des fréquences de contraction des fibres musculaires à la concentration de 10⁻⁵M. Le composé 2 présente une inhibition d'environ 55 % à la concentration de 10⁻⁵M.

### Exemple 3

| Lotion capillaire : | | | % en poids |
|---|---|---|---|
| - | Composé 1 | | 1 |
| - | Propylène glycol | | 30 |
| - | Alcool éthylique | | 40 |
| - | Eau | qsp | 100 |

### Exemple 4

| Mascara cire/eau : | | | % en poids |
|---|---|---|---|
| - | Cire d'abeille | | 6 |
| - | Cire de paraffine | | 13 |
| - | Huile de jojoba hydrogénée | | 2 |
| - | Polymère filmogène hydrosoluble | | 3 |
| - | Stéarate de triéthanolamine | | 8 |
| - | Composé 2 | | 1 |
| - | Pigment noir | | 5 |
| - | Conservateur | qs | |
| - | Eau | qsp | 100 |

Ce mascara s'applique sur les cils comme un mascara classique avec une brosse à mascara.

## Revendications

1. Utilisation cosmétique d'au moins un dérivé de pipéridine de formule (I) ou de l'un de ses sels ou isomères, comme agent pour induire et/ou stimuler la croissance des fibres kératiniques des êtres humains et/ou freiner leur chute et/ou augmenter leur densité, 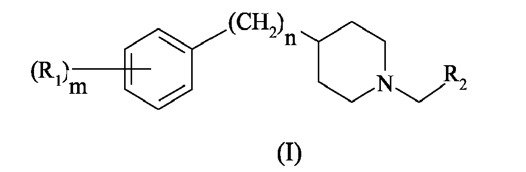 dans laquelle :
- R₁ désigne un halogène ou un radical choisi parmi un radical alkyle linéaire ou ramifié en C₁-C₆, un groupe OR, un groupe NRR', un groupe CF₃, un groupe NHCOR ou un groupe CONRR' ;
- R₂ désigne un radical alkyle ou alkényle linéaire ou ramifié, en C₁-C₂₀, dont la chaîne hydrocarbonée peut être éventuellement interrompue par une fonction - CO- éventuellement substitué par au moins un groupe OR, COOR, NRR', NHCOR ou CONRR' et/ou par un groupe phényle éventuellement substitué par un ou plusieurs radicaux R₁ ;
avec R et R' désignant, indépendamment l'un de l'autre, un atome d'hydrogène ou un radical alkyle en C₁-C₆, linéaire ou ramifié,
- m représente un entier variant de 0 à 5 ; et
- n représente un entier variant de 0 à 5.

2. Utilisation d'au moins un dérivé pipéridine de formule générale (I) telle que définie en revendication 1 ou de l'un de ses sels ou isomères, pour la préparation d'une composition cosmétique de soin et/ou de maquillage des fibres kératiniques d'êtres humains pour réduire la chute des fibres kératiniques et/ou augmenter leur densité.

3. Utilisation d'au moins un dérivé pipéridine de formule (I) telle que définie en revendication 1 ou de l'un de ses sels ou isomères, pour la préparation d'une composition de soin et/ou de traitement des fibres kératiniques d'êtres humains, destinée à induire et/ou stimuler la croissance des fibres kératiniques.

4. Utilisation selon l'une quelconque des revendications précédentes, **caractérisée en ce que** les fibres kératiniques sont des cheveux, des sourcils, des cils, des poils de barbe, de moustache et des poils pubiens.

5. Utilisation d'au moins un dérivé pipéridine de formule générale (I) telle que définie en revendication 1 ou de l'un de ses sels ou isomères, pour la préparation d'une composition cosmétique de soin capillaire d'êtres humains, destinée à traiter l'alopécie andro-chrono-génétique.

6. Utilisation d'au moins un dérivé pipéridine de formule générale (I) telle que définie en revendication 1 ou de l'un de ses sels ou isomères, pour la préparation d'une composition capillaire d'êtres humains, destinée à induire et/ou stimuler la croissance des cheveux.

7. Utilisation d'au moins un dérivé pipéridine de formule générale (I) telle que définie en revendication 1 ou de l'un de ses sels ou isomères, pour la préparation d'une composition cosmétique de soin et/ou de maquillage des cils d'êtres humains, destinée à induire et/ou stimuler la croissance des cils et/ou augmenter leur densité.

8. Utilisation d'au moins un dérivé pipéridine de formule générale (I) telle que définie en revendication 1 ou de l'un de ses sels ou isomères, pour la préparation d'une composition destinée à traiter les désordres liés à une réduction de la microcirculation ou vascularisation cutanée et notamment d'un follicule pileux chez l'être humain.

9. Utilisation selon l'une quelconque des revendications précédentes, **caractérisée en ce que** ledit dérivé pipéridine de formule (I) ou l'un de ses sels ou isomères répond à l'une au moins des conditions suivantes :
- m varie de 0 à 3 et de préférence est égal à 0
- n est égal à 0, 1, 2 ou 3, et de préférence à 0 ou 3,
- R₂ désigne un radical alkyle ou alkényle linéaire ou ramifié, en C₁-C₁₄, dont la chaîne hydrocarbonée peut être interrompue par un motif carbonyle et/ou être substituée par un groupe phényle et/ou une fonction hydroxyle.

10. Utilisation selon l'une quelconque des revendications précédentes, **caractérisée en ce que** ledit dérivé pipéridine de formule (I) est choisi parmi : 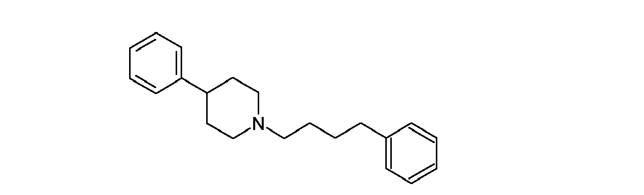 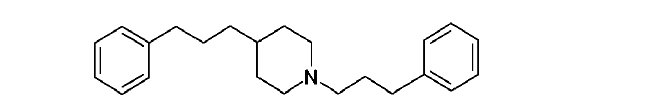 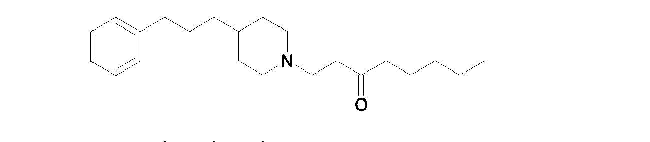 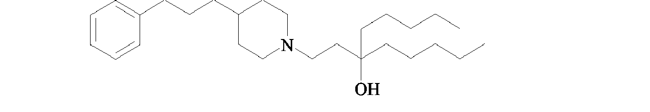 leurs sels, isomères et leurs mélanges.

11. Utilisation selon l'une quelconque des revendications 2 à 10, **caractérisée en ce que** ledit dérivé pipéridine de formule (I) ou un mélange de dérivés pipéridines de formule (I) est utilisé à raison de 10⁻³ à 10 %, de préférence de 10⁻² à 5 % en poids, par rapport au poids total de la composition.

12. Utilisation selon l'une quelconque des revendications précédentes, **caractérisée en ce que** ledit composé de formule générale (I) est formulé dans une composition destinée à une application topique.

13. Utilisation selon la revendication précédente, **caractérisée en ce que** la composition se présente sous forme de crème ou lotion capillaire, de shampooing, d'après-shampooing, de mascara capillaire ou pour cils.

14. Utilisation selon la revendication 13, **caractérisée en ce que** la composition est sous forme d'une solution ou suspension aqueuse, alcoolique ou hydro-alcoolique.

15. Utilisation selon l'une quelconque des revendications 2 à 14, **caractérisée en ce que** la composition contient en outre au moins un ingrédient choisi parmi les solvants, les épaississants ou gélifiants de phase aqueuse ou de phase huileuse, les matières colorantes solubles dans le milieu de la composition, les charges, les pigments, les antioxydants, les conservateurs, les parfums, les électrolytes, les neutralisants, les polymères filmogènes, les agents bloqueurs d'U.V., les actifs cosmétiques et pharmaceutiques autres que les composés de formule générale (I) et leurs mélanges.

16. Utilisation selon l'une quelconque des revendications 1 à 15, **caractérisée en ce que** ledit composé de formule générale (I) est associé à au moins un composé additionnel actif favorisant la repousse et/ou limitant la chute des fibres kératiniques.

17. Utilisation selon la revendication précédente, **caractérisée en ce que** le composé actif additionnel est choisi parmi l'aminexil, le 6-0-[(9Z,12Z)-octadéca-9,12-diènoyl]hexapyranose, les inhibiteurs de lipoxygénase, les inhibiteurs de bradykinine, les prostaglandines et leurs dérivés, les agonistes ou antagonistes des récepteurs des prostaglandines, les analogues non prostanoïques de prostaglandines, les vasodilatateurs, les antiandrogènes, les cyclosporines et leurs analogues, les antimicrobiens, les anti-inflammatoires, les rétinoïdes, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryle substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotérines, les cytokines et les promoteurs de facteurs de croissance, les inhibiteurs d'IL-1 ou d'IL-6, les promoteurs d'IL-10, les inhibiteurs de TNF, les vitamines, les benzophénones, l'hydantoïne, l'acide rétinoïque, les agents antiprurigineux, les antiparasitaires, les antifongiques, les agents antagonistes de calcium, les hormones, les triterpènes, les agents antiandrogènes, les inhibiteurs stéroïdiens ou non stéroïdiens des 5-α-réductases, les agonistes de canaux potassiques, les antagonistes du récepteur FP, leurs mélanges.

18. Utilisation selon l'une quelconque des revendications précédentes, **caractérisée en ce que** ledit composé est associé à au moins un actif choisi parmi les protéines, les hydrolysats de protéine, les acides aminés, les polyols, l'urée, l'allantoïne, les sucres et dérivés de sucre, les vitamines hydrosolubles, les extraits végétaux, les hydroxy-acides, le rétinol, le tocophérol, les dérivés du rétinol ou du tocophérol, les acides gras essentiels, les céramides, les huiles essentielles, les dérivés de l'acide salicylique comme le n-octanoyl-5 salicylique, les esters des hydroxy-acides, les phospholipides, leurs mélanges.

19. Procédé cosmétique de traitement des fibres kératiniques humaines et/ou de la peau d'où émergent lesdites fibres, afin de stimuler la croissance des fibres kératiniques humaines, **caractérisé en ce qu'**il comprend au moins l'application sur les fibres kératiniques humaines et/ou la peau, d'une composition cosmétique comprenant une quantité efficace d'au moins un dérivé pipéridine de formule générale (I) telle que définie en revendications 1 et 9 ou 10, ou de l'un de ses sels ou isomères, à laisser celle-ci en contact avec les fibres kératiniques et/ou la peau d'où émergent les fibres, et éventuellement à rincer les fibres kératiniques et/ou la peau.

20. Procédé cosmétique de traitement de l'alopécie andro-chrono-génétique, **caractérisé en ce qu'**il consiste à appliquer sur les cheveux humains et/ou le cuir chevelu, une composition cosmétique comprenant une quantité efficace d'au moins un dérivé pipéridine de formule générale (I) tels que définis en revendications 1 et 9 ou 10, ou de l'un de ses sels ou isomères, à laisser celle-ci en contact avec les fibres kératiniques et/ou la peau, et éventuellement le rinçage des fibres kératiniques et/ou la peau.

21. Procédé cosmétique de soin et/ou de maquillage des cils, **caractérisé en ce qu'**il comprend au moins l'application sur les cils et/ou les paupières supérieures, d'une composition de mascara contenant au moins un dérivé pipéridine de formule générale (I) ou l'un de ses sels ou isomères tels que définis en revendications 1 et 9 ou 10.
